# FASCICULE DE BREVET EUROPEEN

(11) **EP 0 943 310 B1**
(45) Date de publication et mention de la délivrance du brevet: **06.03.2002**
(21) Numéro de dépôt: 99400284.8
(22) Date de dépôt: 08.02.1999
(51) Int. Cl.: A61K 7/043, A61K 7/48

(54) **Composition filmogène comprenant un polyurethane en dispersion aqueuse et un agent plastifiant**
Filmbildende Zusammensetzung enthaltend eine wässrige Polyurethandispersion und einen Weichmacher
Film-forming composition comprising an aqueous polyurethane dispersion and a plasticizer

(30) Priorité: 09.03.1998 FR 9802838
(43) Date de publication de la demande: 22.09.1999
(73) Titulaire: L'OREAL, 75008 Paris (FR)
(72) Inventeur: De La Poterie, Valérie, 77820 Le Chatelet en Brie (FR)
(74) Mandataire: Lhoste, Catherine

(56) Documents cités:
- EP-A- 0 613 676
- EP-A- 0 637 600
- EP-A- 0 679 384
- EP-A- 0 740 933
- EP-A- 0 775 483
- DATABASE WPI Week 9125 Derwent Publications Ltd., London, GB; AN 91-183195 XP002089907 "Durable manicuring material - contg. nitrocellulose, with e.g. dimethyl phthalate, triethyl phthalate etc. , and ethanol solvent" & JP 03 112916 A (KANEBO), 14 mai 1991

## Description

L'invention a pour objet une composition filmogène, notamment topique, comprenant un polyuréthane filmogène destinée en particulier aux domaines cosmétique et/ou dermatologique. L'invention se rapporte aussi à une utilisation de cette composition pour le traitement et le soin des matières kératiniques telles que la peau, les ongles, les cils, les sourcils, les cheveux ou des muqueuses telles que les lèvres et l'intérieur des paupières. Elle est destinée plus spécialement au traitement et/ou au soin des ongles.

Il est connu des demandes EP-A-143480, EP-A-648485 et EP-A-EP-A-775483 des compositions de maquillage des ongles et des lèvres comprenant comme polymère filmogène un polyuréthane en dispersion aqueuse.

Le film déposé sur l'ongle ou les lèvres après l'application d'une composition filmogène doit présenter une bonne flexibilité pour éviter son craquèlement ou son écaillement. Dans ce but, il est d'usage courant d'ajouter dans la composition filmogène des agents plastifiants permettant de régler la flexibilité du film sans affaiblir sa résistance physique.

Le document EP-A-637600 décrit des compositions de vernis à ongles comprenant une dispersion aqueuse de polyester-polyuréthane et un solvant plastifiant. Il est également connu des documents EP-A-613676 et JP-A-03112916 des vernis en milieu solvant organique contenant un citrate.

Par ailleurs, les compositions filmogènes tels que les vernis à ongles doivent posséder certaines caractéristiques rhéologiques, telle qu'une faible viscosité, pour faciliter leur application, notamment à l'aide d'un pinceau. Dans ce but, il est souhaitable que la composition n'épaississe pas dans le flacon au cours du temps et donc reste stable pendant toute la durée du stockage.

Or, la demanderesse a constaté que des compositions comprenant une dispersion aqueuse de polyuréthane et certains agents plastifiants ont tendance à épaissir au cours du temps. Cette augmentation de la viscosité se produit plus particulièrement lors du stockage de la composition à température élevée, en particulier à partir de 45 °C, et notamment au bout d'une semaine, ou bien plusieurs mois à température ambiante. La composition ainsi épaissie est difficile à appliquer et ne permet pas d'obtenir le dépôt d'un film homogène et continu. Certains agents plastifiants conduisent même à une prise en masse dans le flacon rendant la composition inutilisable.

Le but de la présente invention est de proposer une composition filmogène contenant une dispersion aqueuse de particules de polyuréthane présentant de bonnes propriétés de stabilité et/ou cosmétiques et ne présentant pas les inconvénients mentionnés ci-dessus.

La demanderesse a découvert qu'une telle composition pouvait être obtenue en utilisant une sélection d'agents plastifiants particuliers. Ces agents plastifiants permettent de stocker la composition à température élevée, notamment à 45 °C, pendant 2 semaines voire plusieurs mois, tout en évitant une augmentation importante de la viscosité de la composition au cours du temps. On obtient alors une composition filmogène présentant une viscosité stable dans le temps. La composition est facile à appliquer, notamment à l'aide d'un pinceau, sur le support à traiter comme par exemple les matières kératiniques.

La présente invention a pour objet une composition filmogène comprenant au moins une dispersion aqueuse de particules de polyuréthane et au moins un agent plastifiant, caractérisée par le fait que l'agent plastifiant est choisi parmi les solvants présentant un paramètre moyen de solubilité dH de HANSEN à 25°C tel que dH ≤ 8 (J/cm³)^{½}.

De préférence, l'agent plastifiant selon l'invention peut présenter également un paramètre moyen de solubilité dP de HANSEN à 25°C tel que dP ≤ 3 (J/cm³)^{½}, et mieux dP ≤ 2 (J/cm³)^{½}.

La définition des solvants dans l'espace de solubilité tridimensionnel selon HANSEN est décrite dans l'article de C. M. HANSEN : "The three dimensionnal solubility parameters" J. Paint Technol. 39, 105 (1967) ;
- dH caractérise les forces d'interactions spécifiques (type liaisons hydrogène, acide/base, donneur/accepteur, etc...) ;
- dP caractérise les forces d'interactions de DEBYE entre dipôles permanents ainsi que les forces d'interactions de KEESOM entre dipôles induits et dipôles permanents.

Les paramètres dP et dH sont exprimés en (J/cm³)^{½}.

Selon l'invention, l'agent plastifiant peut être choisi parmi les adipates de dialkyle en C3-C10, les phtalates de dialkyle en C3-C10, l'acétyl tributyle citrate.

De préférence, l'agent plastifiant peut être choisi parmi le phtalate de dibutyle (dH = 7,5 ; dP = 2,8), le phtalate de diéthyle -2-hexyle (dH = 5,92 ; dP = 1,76), l'adipate de diisopropyle (dH = 7,76 ; dP = 2,98), l'adipate de dibutyle (dH = 7,28 ; dP = 2,63), l'adipate de diéthyl-2-hexyle (dH = 5,97 ; dP = 1,76), l'acétyl citrate de tributyle (dH = 7,09 ; dP = 1,75).

Préférentiellement, l'agent plastifiant peut être choisi parmi le phtalate de dibutyle, l'adipate de diisopropyle, l'adipate de diéthyl-2-hexyle, l'acétyl citrate de tributyle.

Le polyuréthane en dispersion aqueuse peut être un polyuréthane anionique. Ce caractère anionique est notamment dû à la présence de groupements à fonction acide carboxylique ou acide sulfonique dans le polymère.

Selon l'invention, on peut utiliser une ou plusieurs dispersions aqueuses d'un ou plusieurs polyuréthanes.

Le polyuréthane peut être choisi parmi les polyester-polyuréthanes et les polyéther-polyuréthanes, et de préférence parmi les polyester-polyuréthanes anioniques.

Avantageusement, la dispersion aqueuse de polyuréthane polyuréthane peut être être choisi parmi celles dont la taille des particules de polyuréthane va de 2 à 100 nm et dont la dureté d'un film obtenu après séchage, durant 24 heures à 30 °C et à 50 % d'humidité relative, d'une couche de 300 µm d'épaisseur (avant séchage) d'une dispersion aqueuse à 28 % de matière sèche desdites particules de polyuréthane va de 15 à 300 secondes.
La dureté du film est mesurée selon la norme ASTM D-43-66, ou la norme NF-T 30-016 (octobre 1981), à l'aide d'un pendule de Persoz.

Comme polyuréthane utilisable selon l'invention, on peut notamment citer les polyester-polyuréthanes vendus sous les dénominations "AVALURE UR-405® ", "AVALURE UR-425® ", "SANCURE 2060® " par la société SANNCOR et les polyéther-polyuréthanes vendus sous les dénominations "SANCURE 878® " par la société SANNCOR, "NEOREZ R 970® " par la société ICI.

Selon l'invention, le polyuréthane peut être présent dans la composition en une quantité allant de 3 à 50 % en poids par rapport au poids total de la composition, et de préférence de 10 % à 35 % en poids.

Dans la composition selon l'invention, l'agent plastifiant peut être présent en une quantité allant de 0,1 % à 20 % en poids, par rapport au poids total de la composition, et de préférence de 0,5 % à 10 % en poids.

Par ailleurs, la composition selon l'invention peut contenir des adjuvants couramment utilisés dans les compositions cosmétiques, notamment topiques. On peut citer à titre d'exemple d'adjuvants les colorants, les pigments, les nacres, les laques, les agents anti-UV, les conservateurs, les agents épaississants, les tensioactifs, les cires, les parfums, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut être avantageusement utilisée pour le traitement, le maquillage, ou le soin des matières kératiniques et/ou des muqueuses selon la nature des actifs utilisés. La composition de maquillage peut être un vernis à ongles, un eye-liner, un mascara, un fond de teint, un anti-cernes, un fard à paupières ou à joues, un rouge à lèvres (laque à lèvres), ou bien encore une composition de maquillage du corps.

La composition selon l'invention peut avantageusement se présenter sous forme d'un vernis à ongles ou d'une composition de soin des ongles.

L'invention se rapporte aussi à un procédé de traitement cosmétique ou de maquillage des matières kératiniques, et notamment des ongles, et/ou des muqueuses, consistant à appliquer sur les matières kératiniques et/ou les muqueuses une composition telle que décrite précédemment.

L'invention a également pour objet l'utilisation d'au moins une dispersion aqueuse de particules de polyuréthane et d'au moins un agent plastifiant choisi parmi les solvants présentant un paramètre moyen de solubilité dH de HANSEN à 25°C tel que dH ≤ 8 (J/cm³)^{½} tels que définis précédemment pour l'obtention d'une composition filmogène présentant une viscosité stable dans le temps et/ou facile à appliquer et/ou ne prenant pas en masse dans le temps.

On va maintenant donner des exemples illustrant la présente invention sans toutefois la limiter.

### Exemples 1 à 8 : comparatifs

On a préparé 3 compositions (E1 à E3) selon l'invention et 5 compositions (E4 à E8) ne faisant pas partie de l'invention ayant la constitution suivante :
- Dispersion aqueuse de polyester-polyuréthane (AVALURE UR-405® ) 35 g MA
- plastifiant 3,5 g
- eau qsp 100 g
en utilisant différents plastifiants à une teneur de 10 % en poids par rapport au poids de polymère.

Ces compositions ont été stockées à 45 °C pendant 2 mois. On a mesuré la viscosité de chaque composition à quatre moments différents durant le stockage:
T0 = temps initial du mélange
T1 = T0 + 1 semaine
T2 = T0 + 1 mois
T3 = T0 + 2 mois

Les viscosités ont été mesurées à l'aide du viscosimètre BROOKFIELD RVTDV 2+ avec le mobile RV6 à une vitesse de rotation de 100 tours/minute, après 10 minutes de rotation du mobile, à 25 °C.

On a obtenu les résultats suivants, les viscosités étant exprimées en mPa. s.

| **Plastifiant** | **T0** | **T1** | **T2** | **T3** |
|---|---|---|---|---|
| phtalate de dibutyle **(E1)** | 864 | 989 | 1040 | 1440 |
| adipate de diéthylhexyle **(E2)** | 659 | 682 | 611 | 605 |
| acétyle citrate de tributyle **(E3)** | 957 | 906 | 752 | 656 |
| citrate de triéthyle * **(E4)** | 224 | trop épais | prise en masse | prise en masse |
| TPn Bu * **(E5)** | 704 | 1280 | 4740 | 32300 |
| lactate d'éthyle ***(E6)** | 122 | prise en masse | prise en masse | prise en masse |
| Arcosolv PTB* **(E7)** | 330 | 595 | 1550 | 5440 |
| Pn Bu * **(E8)** | 650 | 7420 | 25100 | trop épais |

| | | | | |
|---|---|---|---|---|
| * plastifiants ne faisant pas partie de l'invention: - citrate de triéthyle (dH = 13,39 ; dP = 4,3) - TPn BU : n-butyl éther de tripropylène glycol (dH = 10,8; dP = 3,4) - Arcosolv PTB : t-butyl éther de propylène glycol (dH = 12,52 ; dP = 4,37) - Pn Bu : n-butyl éther de propylène glycol (dH = 12,6 ; dP = 4,4) | | | | |

Les résultats obtenus montrent que les plastifiants selon l'invention permettent d'obtenir une composition filmogène, après stockage à 45 °C, ayant une viscosité au plus de 1380 mPa.s nettement inférieure aux viscosités obtenues pour les compositions comprenant un plastifiant ne faisant pas partie de la présente invention, ces dernières conduisant même à la formation de stick. Ainsi, seules les compositions selon l'invention restent fluides après le stockage et sont aptes à être appliquées sur un support à traiter comme les ongles ou les lèvres elles présentent une viscosité stable dans le temps.

### Exemples 9 à 13 : comparatifs

De la même façon qu'à l'exemple 1, on a préparé 4 compositions (E9 à E12) selon l'invention et 1 composition (E13) ne faisant pas partie de l'invention avec le même polymère filmogène en utilisant différents plastifiants à une teneur de 5 % en poids (au lieu de 10 % en poids) par rapport au poids total de polymère.

On a obtenu les résultats de viscosité suivants exprimés en mPa.s:

| **Plastifiant** | **T0** | **T1** | **T2** | **T3** |
|---|---|---|---|---|
| adipate de diisopropyle **(E9)** | 573 | 531 | 598 | 685 |
| phtalate de dibutyle **(E10)** | 538 | 573 | 566 | 611 |
| adipate de diéthylhexyle **(E11)** | 634 | 560 | 522 | 509 |
| acétyle citrate de tributyle **(E12)** | 819 | 653 | 579 | 614 |
| citrate de triéthyle * **(E13)** | 208 | 806 | trop épais | prise en masse |

| | | | | |
|---|---|---|---|---|
| * plastifiant ne faisant pas partie de l'invention | | | | |

On a constaté que la composition comprenant le citrate de triéthyle (ne faisant pas partie de l'invention) conduit après 1 mois de stockage à 45 °C à la formation de stick tandis que les compositions selon l'invention restent fluides, même après deux mois de stockage à 45 °C.

### Exemples 14 à 17 : comparatifs

De la même façon qu'à l'exemple 1, on a préparé 3 compositions (E14 à E16) seIon l'invention et 1 composition (E17) ne faisant pas partie de l'invention ayant la constitution suivante :
- dispersion aqueuse de polyester-polyuréthane (SANCURE 2060® ) 27 g MA
- plastifiant 2,7 g
- eau qsp 100 g
la teneur en plastifiant étant de 10 % en poids par rapport au poids de polymère.

Les viscosités ont été mesurées à T0, T1 et T2 définis ci-dessus.

On a obtenu les résultats de viscosité suivants exprimés en mPa.s :

| **Plastifiant** | **T0** | **T1** | **T2** |
|---|---|---|---|
| adipate de diéthylhexyle **(E14)** | 270 | 290 | 270 |
| acétyl citrate de tributyle **(E15)** | 300 | 300 | 330 |
| adipate de diisopropyle **(E16)** | 340 | 400 | 440 |
| TPn Bu * **(E17)** | 540 | 820 | 1010 |

| | | | |
|---|---|---|---|
| * plastifiant ne faisant pas partie de l'invention | | | |

Les résultats obtenus montrent que les compositions selon l'invention ont une viscosité inférieure à celle de la composition comprenant le TPn Bu (ne faisant pas partie de l'invention). En outre, seules les compositions selon l'invention ont une viscosité stable au cours du temps. Ces compositions plus fluides sont donc plus facile à étaler par exemple avec un pinceau que la composition comprenant le TPn Bu.

### Exemples 18 à 21 : comparatifs

De la même façon qu'à l'exemple 1, on a préparé 3 compositions (E18 à E20) selon l'invention et 1 composition (E21) ne faisant pas partie de l'invention ayant la constitution suivante :
- dispersion aqueuse de polyéther-polyuréthane (SANCURE 878® ) 34 g MA
- plastifiant 3,4 g
- eau qsp 100 g
la teneur en plastifiant étant de 10 % en poids par rapport au poids de polymère.

On a obtenu les résultats de viscosité suivants exprimés en mPa.s :

| **Plastifiant** | **T0** | **T1** | **T2** | **T3** |
|---|---|---|---|---|
| adipate de diéthylhexyle **(E18)** | 830 | 580 | 760 | 690 |
| acétyl citrate de tributyle **(E19)** | 760 | 520 | 510 | 350 |
| adipate de diisopropyle **(E20)** | 790 | 470 | 410 | 430 |
| TPn Bu * **(E21)** | 1400 | 1000 | 1230 | 1410 |

| | | | | |
|---|---|---|---|---|
| * plastifiant ne faisant pas partie de l'invention | | | | |

Les résultats obtenus montrent que les compositions selon l'invention ont une viscosité inférieure à celle de la composition comprenant le TPn Bu (ne faisant pas partie de l'invention).

### Exemple 22 :

On a préparé une composition fluide à appliquer sur les lèvres ayant la constitution suivante :
- Dispersion aqueuse de polyester-polyuréthane (AVALURE UR-425® ) 20 g MA
- acétyl citrate de tributyle 1 g
- pigments 4 g
- épaississant 1 g
- conservateurs qs
- eau qsp 100 g

On obtient une composition fluide qui présente une bonne stabilité au stockage et qui s'applique facilement sur les lèvres.

### Exemple 23 :

On a préparé un vernis à ongles ayant la composition suivante :
- dispersion aqueuse de polyester-polyuréthane (AVALURE UR-405® ) 30 g MA
- adipate de diisopropyle 3 g
- pigments 4 g
- épaississant 1 g
- conservateurs qs
- eau qsp 100 g

On obtient un vernis fluide présentant une viscosité stable au stockage et qui s'étale facilement sur les ongles

### Exemple 24 :

On a préparé un eye-liner ayant la composition suivante :
- dispersion aqueuse de polyéther-polyuréthane (SANCURE 878® ) 25 g MA
- adipate de 2-éthyl hexyle 1,25 g
- pigments 13 g
- conservateurs qs
- eau qsp 100 g

On obtient un vernis fluide présentant une bonne stabilité au stockage et qui s'applique facilement sur le bord des paupières.

## Revendications

1. Composition cosmétique filmogène comprenant au moins une dispersion aqueuse de particules de polyuréthane et au moins un agent plastifiant, **caractérisée par le fait que** l'agent plastifiant est choisi parmi les solvants présentant un paramètre moyen de solubilité dH de HANSEN à 25°C tel que dH ≤ 8 (J/cm³)^{½}, l'agent plastifiant étant choisi dans le groupe formé par les adipates de dialkyle en C3-C10, les phtalates de dialkyle en C3-C10, l'acétyl citrate de tributyle, ledit agent plastifiant étant présent en une quantité allant de 0,1 % à 20 % en poids, par rapport au poids total de la composition.

2. Composition selon la revendication 1, **caractérisée par le fait que** l'agent plastifiant présente un paramètre moyen de solubilité dP à 25°C tel que dP ≤ 3 (J/cm³)^{½}.

3. Composition selon l'une quelconque des revendications précédentes, **caractérisée par le fait que** l'agent plastifiant est choisi dans le groupe formé par le phtalate de dibutyle, le phtalate de diéthyle -2-hexyle, l'adipate de diisopropyle, l'adipate de dibutyle, l'adipate de diéthyl-2-hexyle, l'acétyl-citrate de tributyle.

4. Composition selon l'une quelconque des revendications précédentes, **caractérisée par le fait que** l'agent plastifiant est choisi parmi le phtalate de dibutyle, l'adipate de diisopropyle , l'adipate de diéthyl-2-hexyle, l'acétyl citrate de tributyle.

5. Composition selon l'une quelconque des revendications précédentes, **caractérisée par le fait que** le polyuréthane est un polyuréthane anionique.

6. Composition selon l'une quelconque des revendications précédentes, **caractérisée par le fait que** le polyuréthane est choisi parmi les polyester-polyuréthanes et les polyéther-polyuréthanes.

7. Composition selon l'une quelconque des revendications précédentes, **caractérisée par le fait que** le polyuréthane est un polyester-polyuréthane anionique.

8. Composition selon l'une quelconque des revendications précédentes, **caractérisée par le fait que** la taille des particules de polyuréthane va de 2 à 100 nm et que la dureté d'un film obtenu après séchage, durant 24 heures à 30 °C et à 50 % d'humidité relative, d'une couche de 300 µm d'épaisseur d'une dispersion aqueuse à 28 % de matière sèche desdites particules de polyuréthane va de 15 à 300 secondes, la dureté du film étant mesurée à l'aide d'un pendule de Persoz selon la norme ASTM D-43-66 ou la norme NF-T 30-016.

9. Composition selon l'une quelconque des revendications précédentes, **caractérisée par le fait que** le polyuréthane est présent en une quantité allant de 3 à 50 % en poids par rapport au poids total de la composition.

10. Composition selon l'une quelconque des revendications précédentes, **caractérisée par le fait que** ledit agent plastifiant est présent en une quantité allant de 0,5 % à 10 % en poids, par rapport au poids total de la composition.

11. Composition cosmétique selon l'une quelconque des revendications précédentes, **caractérisée par le fait qu'**elle contient en outre au moins un pigment et/ou un colorant.

12. Composition cosmétique selon l'une quelconque des revendications précédentes, **caractérisée par le fait qu'**elle contient en outre au moins un épaississant.

13. Composition cosmétique selon l'une quelconque des revendications précédentes, **caractérisée par le fait qu'**elle se présente sous forme de composition de maquillage.

14. Procédé de traitement cosmétique des matières kératiniques et/ou des muqueuses, **caractérisé par le fait que** l'on applique sur les matières kératiniques et/ou sur les muqueuses une composition selon l'une quelconque des revendications 1 à 13.

15. Procédé de maquillage des matières kératiniques et/ou des muqueuses, **caractérisé par le fait que** l'on applique sur les matières kératiniques et/ou sur les muqueuses une composition selon l'une quelconque des revendications 1 à 13.

16. Utilisation d'au moins une dispersion aqueuse de particules de polyuréthane et d'au moins un agent plastifiant tels que définis selon l'une quelconque des revendications 1 à 10, ledit agent plastifiant étant présent en une quantité allant de 0,1 % à 20 % en poids, par rapport au poids total de la composition, pour l'obtention d'une composition filmogène présentant une viscosité stable dans le temps et/ou facile à appliquer et/ou ne prenant pas en masse dans le temps.

## Patentansprüche

1. Filmbildende kosmetische Zusammensetzung, die mindestens eine wäßrige Dispersion von Polyurethanpartikeln und mindestens einen Weichmacher enthält, **dadurch gekennzeichnet, daß** der Weichmacher unter den Lösungsmitteln ausgewählt ist, die bei 25 °C einen mittleren Solubilitätsparameter dH gemäß HANSEN von dH ≤ 8 (J/cm³)^{1/2} aufweisen, wobei der Weichmacher unter den C₃₋₁₀-Dialkyladipaten, C₃₋₁₀-Dialkylphthalaten und Tributylacetylcitrat ausgewählt ist und in einer Menge von 0,1 bis 20 Gew.-%, bezogen auf das Gesamtgewicht der Zusammensetzung, vorliegt.

2. Zusammensetzung nach Anspruch 1, **dadurch gekennzeichnet, daß** der Weichmacher bei 25 °C einen mittleren Solubilitätsparameter dP von dP ≤ 3 (J/cm³)^{1/2} aufweist.

3. Zusammensetzung nach einem der vorhergehenden Ansprüche, **dadurch gekennzeichnet, daß** der Weichmacher unter Dibutylphthalat, 2-Diethylhexylphthalat, Diisopropyladipat, Dibutyladipat, 2-Diethylhexyladipat und Tributylacetylcitrat ausgewählt ist.

4. Zusammensetzung nach einem der vorhergehenden Ansprüche, **dadurch gekennzeichnet, daß** der Weichmacher unter Dibutylphthalat, Diisopropyladipat, 2-Diethylhexyladipat und Tributylacetylcitrat ausgewählt ist.

5. Zusammensetzung nach einem der vorhergehenden Ansprüche, **dadurch gekennzeichnet, daß** das Polyurethan ein anionisches Polyurethan ist.

6. Zusammensetzung nach einem der vorhergehenden Ansprüche, **dadurch gekennzeichnet, daß** das Polyurethan unter den Polyester-Polyurethanen und Polyether-Polyurethanen ausgewählt ist.

7. Zusammensetzung nach einem der vorhergehenden Ansprüche, **dadurch gekennzeichnet, daß** das Polyurethan ein anionisches Polyester-Polyurethan ist.

8. Zusammensetzung nach einem der vorhergehenden Ansprüche, **dadurch gekennzeichnet, daß** die Größe der Polyurethanpartikel im Bereich von 2 bis 100 nm liegt, und dadurch, daß die Härte eines Films, der nach dem Trocknen während einer Zeitspanne von 24 h bei 30 °C und 50 % relativer Luftfeuchte einer Schicht von 300 µm Dicke einer wäßrigen Dispersion von 28 % Trockensubstanz der Polyurethanpartikel erhalten wird, im Bereich von 15 bis 300 s liegt, wobei die Härte des Films mit einem Persoz-Pendelschlagwerk nach der Norm ASTM D-43-66 oder der Norm NF-T 30-016 bestimmt wird.

9. Zusammensetzung nach einem der vorhergehenden Ansprüche, **dadurch gekennzeichnet, daß** das Polyurethan in einer Menge von 3 bis 50 Gew.-%, bezogen auf das Gesamtgewicht der Zusammensetzung, vorliegt.

10. Zusammensetzung nach einem der vorhergehenden Ansprüche, **dadurch gekennzeichnet, daß** der Weichmacher in einer Menge von 0,5 bis 10 Gew.-%, bezogen auf das Gesamtgewicht der Zusammensetzung, vorliegt.

11. Kosmetische Zusammensetzung nach einem der vorhergehenden Ansprüche, **dadurch gekennzeichnet, daß** sie außerdem mindestens ein Pigment und/oder ein Farbmittel enthält.

12. Kosmetische Zusammensetzung nach einem der vorhergehenden Ansprüche, **dadurch gekennzeichnet, daß** sie mindestens ein Verdickungsmittel enthält.

13. Kosmetische Zusammensetzung nach einem der vorhergehenden Ansprüche, **dadurch gekennzeichnet, daß** sie in Form einer Zusammensetzung zum Schminken vorliegt.

14. Verfahren zur kosmetischen Behandlung von Keratinsubstanzen und/oder Schleimhäuten, **dadurch gekennzeichnet, daß** auf die Keratinsubstanzen und/oder Schleimhäute eine Zusammensetzung nach einem der Ansprüche 1 bis 13 aufgetragen wird.

15. Verfahren zum Schminken von Keratinsubstanzen und/oder Schleimhäuten, **dadurch gekennzeichnet, daß** auf die Keratinsubstanzen und/oder Schleimhäute eine Zusammensetzung nach einem der Ansprüche 1 bis 13 aufgetragen wird.

16. Verwendung mindestens einer wäßrigen Dispersion von Polyurethanpartikeln und mindestens eines Weichmachers nach einem der Ansprüche 1 bis 10, wobei der Weichmacher in einer Menge von 0,1 bis 20 Gew.-%, bezogen auf das Gesamtgewicht der Zusammensetzung, vorliegt, zur Herstellung einer filmbildenden Zusammensetzung, die eine zeitlich stabile Viskosität aufweist und/oder leicht aufzutragen ist und/oder im Laufe der Zeit nicht fest wird.

## Claims

1. Film-forming cosmetic composition comprising at least one aqueous dispersion of polyurethane particles and at least one plasticizer, **characterized in that** the plasticizer is chosen from solvents with an average Hansen solubility parameter dH at 25°C such that dH ≤ 8 (J/cm³)^{½}, the plasticizer being chosen from the group formed by C3-C10 dialkyl adipates, C3-C10 dialkyl phthalates and acetyl tributyl citrate, the said plasticizer being present in an amount ranging from 0.1% to 20% by weight relative to the total weight of the composition.

2. Composition according to Claim 1, **characterized in that** the plasticizer has an average solubility parameter dP at 25°C such that dP ≤ 3 (J/cm³)^{½}.

3. Composition according to any one of the preceding claims, **characterized in that** the plasticizer is chosen from the group formed by dibutyl phthalate, bis (2-ethylhexyl) Phthalate, diisopropyl adipate, dibutyl adipate, bis(2-ethylhexyl) adipate and acetyl tributyl citrate.

4. Composition according to any one of the preceding claims, **characterized in that** the plasticizer is chosen from dibutyl phthalate, diisopropyl adipate, bis(2-ethylhexyl) adipate and acetyl tributyl citrate.

5. Composition according to any one of the preceding claims, **characterized in that** the polyurethane is an anionic polyurethane.

6. Composition according to any one of the preceding claims, **characterized in that** the polyurethane is chosen from polyesterpolyurethanes and polyetherpolyurethanes.

7. Composition according to any one of the preceding claims, **characterized in that** the polyurethane is an anionic polyesterpolyurethane.

8. Composition according to any one of the preceding claims, **characterized in that** the polyurethane particle size ranges from 2 to 100 nm and **in that** the hardness of a film obtained after drying, for 24 hours at 30°C and at 50% relative humidity, a layer 300 µm thick of an aqueous dispersion containing 28% solids of the said polyurethane particles ranges from 15 to 300 seconds, the hardness of the film being measured with the aid of a Persoz pendulum according to standard ASTM D-43-66 or standard NF-T 30-016.

9. Composition according to any one of the preceding claims, **characterized in that** the polyurethane is present in an amount ranging from 3 to 50% by weight relative to the total weight of the composition.

10. Composition according to any one of the preceding claims, **characterized in that** the said plasticizer is present in an amount ranging from 0.5% to 10% by weight, relative to the total weight of the composition.

11. Cosmetic composition according to any one of the preceding claims, **characterized in that** it also contains at least one pigment and/or one dye.

12. Cosmetic composition according to any one of the preceding claims, **characterized in that** it also contains at least one thickener.

13. Cosmetic composition according to any one of the preceding claims, **characterized in that** it is in the form of a make-up composition.

14. Cosmetic treatment process for keratin substances and/or for mucous membranes, **characterized in that** a composition according to any one of Claims 1 to 13 is applied to the keratin substances and/or to the mucous membranes.

15. Make-up process for keratin substances and/or mucous membranes, **characterized in that** a composition according to any one of Claims 1 to 13 is applied to the keratin substances and/or to the mucous membranes.

16. Use of at least one aqueous dispersion of polyurethane particles and of at least one said plasticizer as defined according to any one of Claims 1 to 10, the said plasticizer being present in an amount ranging from 0.1% to 20% by weight, relative to the total weight of the composition, in order to obtain a film-forming composition whose viscosity is stable over time and/or which is easy to apply and/or which does not set to a solid over time.
